# EUROPEAN PATENT SPECIFICATION

(11) **EP 4 279 027 B1**
(45) Date of publication and mention of the grant of the patent: **25.06.2025**
(21) Application number: 23166990.4
(22) Date of filing: 06.04.2023
(51) Int. Cl.: A61F 2/07, A61F 2/92, A61F 2/82, A61L 31/00, A61L 31/14, A61L 31/04, A61L 31/16

(54) **BIOLOGICAL COVER**
BIOLOGISCHE ABDECKUNG
COUVERTURE BIOLOGIQUE

(30) Priority: 29.04.2022 DE 102022110554; 13.06.2022 EP 22178624
(43) Date of publication of application: 22.11.2023
(60) Divisional application: 25177882.5
(73) Proprietor: Cortronik GmbH, 18119 Rostock-Warnemünde (DE)
(72) Inventor: Thoebe, Martin, 18209 Bartenshagen-Parkentin (DE); Podssun-Soldan, Angela, 18059 Papendorf (DE); Rzany, Alexander, 90449 Nürnberg (DE); Momma, Carsten, 18057 Rostock (DE); Müller, Heinz, 18055 Rostock (DE)
(74) Representative: Biotronik Corporate Services SE

(56) References cited:
- US-A1- 2020 054 435
- US-B1- 6 475 232
- US-B2- 9 205 177
- US-B2- 9 770 352
- US-B2- 9 855 371

## Description

The present application relates to a biological cover and a method for producing such a cover. An apparatus for manufacturing such a cover is described as well.

In recent years, there has been an increase in vascular diseases. A steady advancement in medicine as well as medical technology has made it possible to treat a large number of these diseases with drugs and/or vascular interventions (e.g. cardiovascular interventions) in order to enable and/or support the patient's recovery and to adequately prevent the development of further secondary diseases. In the field of vascular interventions, stents are in many cases the means of choice, e.g. to eliminate (local) stenoses or to prevent their formation at an early stage. Such stents can be wire mesh-based, cylindrical devices that can be inserted into a patient's vein or artery during a vascular intervention and brought to a potential stenosis. In many cases, known stent systems are provided with at least one radially dilatable section, whereby stents can be designed to be self-expanding, for example, or the dilatation can be initiated and controlled from outside the patient's body in order to locally dilate a stenosis, if necessary, or to place a stent at a desired location in a blood vessel in order to prevent the formation of a stenosis there. Since a stent is a foreign body in a patient's body, which can lead to unwanted (rejection) reactions of a patient's body, it has been recognized that it may be beneficial to encase a stent to increase its biocompatibility in the patient's body. To increase the biocompatibility of a stent, stents with a cover made of various polymers (e.g., PTFE, PET, silicone, etc.) or even a biological cover (which are based, for example, on cross-linked pericardial material (or other types of tissue, which are preferably of animal origin)) are used (such as on a balloon-expandable stent as a coronary stent graft). In particular, when biological materials are used, it is common practice to stretch them over a stent and, if necessary, to attach them to the stent using surgical suturing techniques. Today, such concepts have been clinically tested and have essentially proven themselves in practical use. However, despite their success to date, they still show some undesirable disadvantages and side effects. The use of polymers as cover can lead to undesirable effects in hemocompatibility/thrombogenicity as a result of general clot activation at the polymeric surfaces. In this regard, it has been noted that a smaller stent diameter (e.g., in the context of small (coronary) vessel diameters) can lead to larger, potentially occurring problems. Another disadvantage is that almost all known coated stents with biological material require storage in a solution, which makes preassembled "ready-to-use" systems (e.g., systems produced in stock and easily stored) almost impossible. Further see to US6475232B1, US 9,205,177 B2 and US 2020/0054435 A1, where covered stents are described. Therefore, it can be summarized that there is a need for improving implant cover, e.g. stent (system) covers. It is an object of the present invention to overcome these adverse effects of implant covers.

This problem is solved by a biological cover of claim 10 and a method for producing such a cover having the features of claim 1. Preferred embodiments are stated in dependent claims and are described in detail below.

A method of making a biological cover for a stent is described. The method comprises providing a first material in a (substantially) cylindrical configuration, wherein the first material is a biological material. Further, the method comprises providing a second material (different from the first material). The second material is disposed between two layers of the first material, where the two layers of the first material are formed by a fold (also referred to as pleat) in the first material (e.g. by folding a portion of the first material protruding along a longitudinal extent of the stent at least partially within itself). Or in other words the first material has a fold in the first material and has a second material arranged within the fold. The first material may have several folds (e.g. 2 or 3) with a second material arranged within each fold.

By introducing the second material into the fold, i.e. between two layers of the first material, it can be achieved that the respective two layers of the first material are not (irreversibly) cross-linked to each other after addition of the cross-linking agent, and thus a movement of the two layers of the first material relative to each other can be maintained, while the first material can have an (increased) dimensional stability, at least partly based on the addition of the cross-linking agent. Thus, for example, the first material can provide a cover that is or can be applied to a stent in a compressed state. When the stent is expanded, the at least two layers (or the fold) of the first material can then unfold, for example, so that no significant internal mechanical stresses are created in the cover in the process. The unfolding of the at least two layers results in a diameter increase of the cylindrical stent. Thus, the fold within the meaning of this application is not to be understood as wrinkles in the first material, which might be there by default. The fold according to application is an intentional overlap of two material layers of the first material. The fold is configured to enlarge the cylinder diameter of the cylindrical configuration of the first material (e.g. biological material).

The first material and the second material may further be pressed onto a mating member external to the cylindrical configuration. In some examples, the pressing is performed only on a portion of the first material.

A crosslinking agent is applied to the biological material. The method may serve to crosslink the first material so that it can be converted from a relatively more flexible state to a relatively less flexible state. The crosslinking may be performed such that overlapping portions of the folds of the first material are permanently bonded together. In some examples, pressing may be performed only in those portions where bonding is to occur. Overall, a (substantially) cylindrical cover can thus be produced, for example, without sewing or seamlessly. In other examples, however, no joining of overlapping sections is necessary. In these examples, the step of pressing can then be omitted, for example (a transfer to a (substantially) cylindrical arrangement can then be made subsequently, for example, by means of an opening and a tab element, as described herein). According to the invention the first material, is a biological material that comprises crosslinkable groups which are crosslinked by use of the crosslinking agent.

The cover may be arranged to completely encase the stent. Alternatively, the biological cover may be provided, for example, such that a longitudinal extent of the biological cover is shorter than a longitudinal extent of the stent, so that only a longitudinal portion of the stent would be covered by the biological cover.

The biological material is a biological material, preferably a biological tissue, suitable for chemical and/or biochemically crosslinking. The biological material may be for example elastin-containing tissue or tissue containing free amino groups - in particular, collagen-containing tissue. Preferred in the context of the invention are amino group-containing, more preferably collagen-containing, biological and/or artificial tissue components, such as e.g. biological pericardial tissue components. The biological tissue may be an autologous, xenogeneic or allogeneic tissue, or combinations thereof. Biological tissue has a degree of organization that lies between cells and a complete organ. In principle, all types of tissue e.g. from non-mammalian or mammalian tissue including human tissue can be used. The biological material or biological tissue that can be used may be pericardial tissue, skin, ligament, connective tissue, tendons, peritoneal tissue, dura mater, tela submucosa, in particular of the gastrointestinal tract, or pleura. Collagen-containing material of internal organs, cornea, blood vessels, heart valve, intestine or intervertebral discs may also be used as biological material or biological tissue. Preferably, collagen-containing tissue may include pericardium, myocardium, connective tissue, tendons, peritoneal tissue, pleura, dura mater, mucosa (tunica mucosa), or tela submucosa, especially of the gastrointestinal tract (e.g., small intestinal submucosa). The collagen-containing material or tissue may be derived from, for example, human, pig (porcine tissue), sheep, goat, horse, crocodile, kangaroo, ostrich, monkey, preferably primate, octopus, rabbit, or cattle (bovine tissue).

Autologous tissue (in medicine) refers to tissue that was isolated from the human or animal body and is to be re-transplanted elsewhere in the same human or animal body (i.e. originating from the same human or animal body or in other words donor and recipient are the same). The autologous tissue to be used comprises chemically and/or biochemically crosslinkable groups. Autologous material can be, for example, the body's own tissue.

Allogeneic tissue (in medicine) refers either to material that was isolated from another human or animal body that is genetically distinct from the human or animal body into which it is transplanted, but which is of the same species. Thus, allogeneic material is material that has been isolated from a human or animal body that is genetically different from the human or animal body into which the implant is to be placed. Allogeneic tissue can be not from the patient itself (but from a genetic different donor of the same species). Allogeneic here also includes hemiallogeneic (genetically different because of being derived from one parent of the same species and one parent from another species). The allogenic tissue to be used comprises chemically and/or biochemically crosslinkable groups.

Xenogeneic tissue (in medicine) refers to tissue that was isolated from a human or animal body of a different (heterologous) species. Thus, xenogeneic (also known as xenogenous or xenogenic) tissue is material that was isolated form a human or animal body which is different from the human or animal body where the implant is to be implanted. Xenogeneic tissue may also refer to tissue based on human or animal donor cells (cells obtained from a or the human or animal donor) being cultivated in a bioreactor or being obtained via 3D printing. The xenogeneic material, e.g. tissue, can be in its native form, in a fixed form, in a processed form or can comprise combinations thereof.

Preferably, the biological material may comprise a pericardial material. However, it is additionally or alternatively also possible to manufacture the biological material generally from a tissue and/or material with a predominantly collagen-containing matrix (e.g. intestinal tissue, tunica, mucosa, etc.). In this regard, the biological material used may be derived from, for example, animals, e.g., pigs, cattle, sheep, etc. Preferably, the biological material used may be in a sufficiently large (i.e., having a sufficiently large surface area to allow the material used to encase a stent), contiguous, planar, substantially two-dimensional structure. The biological material may be, for example, web-like. It may be a flexible biological material, e.g., a biological tissue, whose thickness is (substantially significantly) thinner than its lateral dimensions.

In some examples, the biological material is (substantially) entirely biological. However, it is also possible for the biological material to have non-biological components, but with the biological component(s) being the majority by weight. The biological material can be provided in a native (stabilized) dried state. It may have a thickness of less than 200 µm, less than 100 µm, e.g. less than 70 µm.

An advantage of such a manufacturing process can be seen, for example, in the fact that the second material can be provided in such a way that it supports the crosslinking process. For example, it may be designed to receive the crosslinking agent and thus act as a kind of sponge during pressing. In particular, exerting pressure on the first material and the second material at least partially applied thereto may initiate "squeezing" of the crosslinking agent out of the first material (similar to drainage), which may result in a corresponding release of the crosslinking agent at the site where the pressure is exerted (and preferably at the site of the desired crosslinking). This can advantageously support the crosslinking process and lead to a faster completion.

The second material is a material (e.g. organic polymer) which cannot be crosslinked to the first material by use of the crosslinking agent. The second material may comprise, e.g., a fabric, e.g., polyester fabric. The second material may comprise, for example, (substantially) non-biological material. The second material may be configured to be removed from the first material (e.g., the biological material), both before and after cross-linking, without leaving any residue. In this regard, the second material may be made of a material that may be a permeable membrane for water and/or a crosslinking agent.

A further advantage of such a manufacturing process of a biological cover of a stent can be seen in providing an initially (essentially) two-dimensional biological cover, which can be transformed into a three-dimensional shape to cover the stent therewith. In this regard, it is an aspect of the present invention not only to be able to encase the stent in a cylindrical shape, but also to be able to deviate from this cylindrical arrangement to allow dilatation of a dilatable stent (e.g., in a radial direction) without a mechanical stress (e.g., caused by a tension force that could cause partial or complete tearing of the biological encasement) of the biological encasement. Preferably, this may also enable the provision of a biological cover of a stent that can be both dilated and converted to a non-dilated state without causing damage to the biological cover.

Further, it is possible to provide a stent with at least one mechanical fastening element to mechanically fix the first material to the stent (e.g., by the crosslinking initiated by the crosslinking agent and the pressing).

The mechanical fastening element may be attached to an outer surface of the stent. The mechanical fixation element may be in addition to a regular wire mesh of the stent. The mechanical fastening element may be integrally made with or attached (removably) to the stent. The mechanical fastening element may be provided as a mandrel-shaped fastening structure (which may extend orthogonally from the stent, for example). It may further be provided that the mandrel-shaped attachment structure may be transformed into a hook-shaped attachment structure by bending. Alternatively, the attachment structure may be provided prefabricated as a hook-shaped attachment structure. Additionally or alternatively, the mechanical fastening element may be formed as a roughened surface of the outer surface of the stent (e.g., by sandblasting the surface of the stent and/or by an indentation, etc.). The mechanical fastening element (e.g., when configured as a roughened surface) may be provided locally on the stent or may extend over an entire outer surface of the stent.

Providing the stent with a mechanical attachment structure provides an additional, mechanical means of attaching the biological coating to the stent, thereby providing additional adhesion of the biological coating to the stent (e.g. by pressing the first material (and thus preferably its collagen fibers) into the mechanical fastening element while pressing the first material against the counter element in the presence of the crosslinking agent) and to prevent an undesired displacement of the biological cover on the stent (e.g. along its longitudinal axis) or a detachment from the stent without having to resort to surgical suturing procedures. For the sake of completeness, it should be further noted that aspects of the present invention do not, however, preclude the use of surgical suturing techniques.

One aspect of the disclosure, not corresponding to the wording of the claims, relates to a method of making a biological cover for a stent. The method may comprise providing a biological material and creating a tab element in the biological material. Further, the method may comprise creating an opening in the biological material for the tab element to pass through.

In particular, the method may be combined with other process steps mentioned herein for the production of a biological coating for a stent.

The opening may be created by an incision (e.g. a slot) in the biological material, for example, along a longitudinal extent of the biological material. The opening may be centered (with respect to a side length) along a side along the longitudinal direction of the biological material. However, the width of the opening may also be adapted to a thickness of the biological material. For example, the width of the opening may be selected to be (substantially) of the order of the thickness of the biological material. The length of the opening may be adapted to a lateral dimension of the tab element, for example (substantially) corresponding to such a dimension, such that the tab element may (substantially) provide a positive fit when passed through the opening.

The tab element may have a relatively narrow section whose lateral dimension is adapted to the length of the opening. Its width may, for example, be adapted to (e.g., substantially correspond to) a thickness of the biological material. Thereafter, a section with a relatively larger lateral dimension may adjoin the outer side of the tab element. This section may, for example, prevent the tab element from slipping back out of the opening. The relatively narrow section can be obtained, for example, by appropriately cutting biological material from a pre-cut section that is, for example, rectangular. The relatively widened section may thereby be obtained, for example, by cutting less material from the rectangular precut, or may correspond, for example, to the corresponding original section of the rectangular precut. After passing the tab element through the opening, the biological material of the tab element may be (substantially) positively received by the opening, for example. The relatively narrow section of the tab element may, for example, form an at least partial positive fit with the opening.

Providing the biological material with a tab element as well as with an opening may enable advantageous and quickly applicable mechanical attachment of the tab element in the opening, thereby enabling a contribution to a dimensionally stable biological cover for a stent.

The opening and/or the tab element can be created such that the biological material can be converted into a (substantially) cylindrical shape by passing it through. The tab element and the opening may be created on two opposite sides, preferably along the longitudinal direction of the biological material, wherein the two opposite sides may be the sides that are brought together to form the (substantially) cylindrical arrangement.

The use of conventional covers for a stent, using material already cross-linked with glutaraldehyde, often required a comparatively costly conventional joining technique by manual suturing of the biological material, using a conventional surgical suture. This was particularly considered to be detrimental to a preferentially (cost) efficient manufacturing process. The mechanical fixation described herein, on the other hand, does not require suturing of the biological material (for its fixation) which may reduce the complexity (and associated manufacturing costs) of the manufacturing process.

First, passing the tab element through the opening can provide mechanical stability, e.g., of the corresponding cylindrical configuration, even before the biological material is crosslinked. This can facilitate handling during preparation and execution of crosslinking. At the same time, the tab element and opening can increase the mechanical stability of the configuration even after crosslinking and reduce the risk of detachment of e.g. the crosslinked sections of the tab element and the surrounding area of the opening (e.g. compared to an configuration in which only overlapping sections are crosslinked without passing a tab element through an opening).

However, it is also possible to crosslink the biological material without passing the tab element through the opening beforehand. In this case, the crosslinked material can then be easily transferred to a cylindrical arrangement by passing the tab element through the opening.

The opening and/or the tab element may be created such that the tab element and the opening may be movable relative to each other so that a diameter of the cylindrical shape may be varied. The opening and/or the tab element may be configured such that the diameter of the cylindrical shape may be (reversibly) increased upon dilatation of the stent. This can be achieved, for example, by making the relatively narrow portion of the tab element significantly longer than the thickness of the biological material. The tab element can then slide through the opening, for example, so that different diameters can be provided.

Providing the opening and/or the tab element in this way can allow the biological cover to be adapted to the current application. For example, the crosslinking of the biological material can be done in such a way that the mobility between the opening and the tab element is provided even after crosslinking. In particular, this may allow the diameter to adapt to a state of dilatation of the stent while avoiding damage to the biological material (e.g., tearing). However, it may also allow the biological material to be adjusted to a specific diameter, for example, prior to crosslinking by means of the orifice and tab element, and then crosslinked at that diameter.

The method may comprise the step of passing the tab element through the opening such that a (substantially) cylindrical configuration of the biological material is produced.

A (substantially) cylindrical configuration may concentrically receive a stent therein.

By passing the tab element through the opening, a dimensionally stable, (substantially) cylindrical arrangement of the biological cover can be obtained. In this state, the biological cover can be kept dimensionally stable until it is used (e.g., attached to a stent) (e.g., as a "ready-to-use" system), which may allow independent fabrication of the stent and biological cover. This may promote simplified manufacture of the biological cover and may allow biological covers to be produced in stock and stored until their immediate use, which may help to ensure sufficient and rapidly usable biological covers.

Crosslinking of the biological material may occur before or after the tab element is passed through the opening. The crosslinking may be performed as a chemical crosslinking. Chemical crosslinking may be performed using a crosslinking agent, such as glutaraldehyde, and the application of pressure. Cross-linking of the biological material prior to passing the tab element through the opening may contribute to increased dimensional stability of the biological material in its initial two-dimensional form, thereby facilitating passage of the tab element through the opening to create a (substantially) cylindrical configuration. This may allow for simplified configuration of the biological material to the stent, as in this case the biological material may have already assumed a degree of dimensional stability. Crosslinking after passing the tab element through the opening may assist in the stability of the biological material (in a preferably (substantially) cylindrical arrangement) as explained, such that the biological material may be obtained in a dimensionally stable and (substantially) cylindrical arrangement.

One aspect of the present disclosure, not corresponding to the wording of the claims, relates to a method of attaching a biological cover to a stent, which method may comprise providing a biological material having a main body and at least one attachment portion protruding away from the main body. Further, the method may comprise encasing the stent with the biological material into a (substantially) cylindrical configuration and folding the at least one attaching portion protruding away into an opening of the stent. In particular, the method may be combined with other process steps mentioned herein for the production of a biological coating for a stent.

In some embodiments, the stent may comprise a wire mesh having at least one opening on the exterior thereof.

The at least one attachment portion protruding away from the main body may be integrally connected to the main body. In an alternative embodiment, it may be provided that the at least one attachment portion is attached to the main body (e.g., by cross-linking). The attachment portion protruding away from the main body may be disposed on a side of the biological material that is transverse to a longitudinal extent of the stent (e.g., when the biological material has been attached to the stent). In some embodiments, it may be provided that the main body is provided with at least two attachment portions protruding from the main body, wherein the at least two attachment portions are attached to the main body on two opposite sides of the main body.

By folding the at least one protruding attachment portion into an opening of the stent, an overlap of two superimposed layers of the biological material can be created. Subsequent cross-linking of the overlap can further create a closed tab element, which can provide mechanical fixation of the biological material to the stent.

Providing the biological material with a main body and at least one attachment portion protruding away from the main body may enable simplified attachment of the biological material to the stent (e.g., without a further suturing step), thereby reducing manufacturing complexity and associated manufacturing costs. Further, such an embodiment of the biological material also enables rapid attachment of the biological material to the stent in a clinical setting. **In** particular, the cover and the stent can be seamlessly joined.

Another aspect relates to a stent having a cover attached to the stent using the methods described herein. In particular, the stent may be an expandable stent, e.g., a self-expanding stent or one that may be expanded by a balloon catheter.

Another aspect of the invention relates to a biological material for a cover of a stent. The biological material comprises a (substantially) cylindrical arrangement having a first cylinder radius and/or diameter, wherein in at least a portion thereof an overlap of the biological material is provided which is configured to at least partially unfold upon radial dilatation of the stent, such that enlargement of the cylindrical arrangement to a second cylinder radius and/or diameter may occur. It should be noted, this aspect may also be combined with features presented in connection with other aspects of the present invention. For example, the biological material may be produced by a process described herein.

The biological cover may be adapted to be attached to a dilatable balloon catheter so as to allow an increase in the size of the cylindrical configuration from a first cylinder radius to a second cylinder radius.

The biological material can be provided in a cross-linked state and thus be applied to the stent as a cover in a cross-linked state. Alternatively, it is also possible to provide the biological material in a non-crosslinked state in the (substantially) cylindrical arrangement and thus crosslink the biological material directly on the stent. In any of the aforementioned applications, the biological material may be prepared as described herein.

By providing the biological material with an overlap present in at least a partial area, it can be achieved that the biological material can follow an expansion of a (dilatable and/or expandable) stent without damage, which can prevent associated complications during a vascular intervention and increase the lifetime of the biological material. Furthermore, the previously discussed cross-linking options (before or after application of the biological material) can allow for individualized adaptation of the biological material to the intended application. In the case of crosslinking the biological material before application of the biological material to the stent, a dimensionally stable, (essentially) cylindrical arrangement can already be obtained, and the biological material can already be stored in a dimensionally stable manner until its application to the stent. Crosslinking only after the biological material has been applied to the stent may allow for an immediate precise-fit application of the biological material to the stent, so that precise, precise-fit pre-dimensioning of the biological material (e.g., to the exact dimensions of the stent) may not be required, which may generally simplify the manufacturing process.

The possibility of at least partial unfolding of the cover can also prevent tearing of the cover. Furthermore, the formation of undesirable recoil forces in the biological cover can be prevented, which can undesirably lead to the cover effectively assuming a cylinder radius that is smaller than the first cylinder radius when it is returned to the first cylinder radius. This can lead to an undesirable reduction in the diameter of the stent (after an already dilated cover has been returned to a non-dilated state) due to the restoring forces acting on the cover.

Another aspect, not corresponding to the wording of the claims, relates to a biological material for a cover of a stent, which may comprise a tab element and an opening for passage of the tab element. The biological material may be provided, for example, by a method described herein.

Features of this aspect may also be combined with features presented in connection with other aspects of the present disclosure.

The biological material including the tab element and the opening may be present as a (substantially) two-dimensional member. For example, a thickness of the biological material as described herein may be (substantially) less than a side length of the biological material. Alternatively, provision may be made to pass the tab element through the opening, thereby converting the biological material to a 3-dimensional configuration. It should be noted that the biological material may be provided in either a crosslinked form or a non-crosslinked form.

Such a configuration of the biological material makes it possible to provide the biological material for a cover of a stent in different, prefabricated designs as a "ready-to-use" product. Thus, it may be possible to store the two-dimensional embodiment of the biological material in a comparatively space-saving manner (e.g., compared to a (substantially) cylindrical configuration, which may be obtained by passing the tab element through the opening), thereby reducing distribution complexity. By providing the biological material in cross-linked or non-cross-linked form, adaptation of the biological material to the particular application can also be achieved in this application as already described herein.

The tab element may be configured such that it is movable relative to the opening along the feedthrough direction after passing through the opening. The tab element and the opening can be provided in such a way that a bidirectional movement of the tab element relative to the feed-through direction can be enabled.

By allowing the tab element to move along a feedthrough direction, a variation of the diameter of the cover can be achieved, which can lead to both an increase and a decrease of the diameter of the cover. This can help to individualize the cover for different vascular interventions, and the biological material can also, for example, immediately follow an expansion of the stent to a required diameter. In the case of using the biological material in a non-crosslinked state, it may be possible to pass the tab element through the opening until a desired diameter of the covering (in (substantially) a cylindrical configuration) is achieved. Subsequently, cross-linking of the biological material can be performed, thereby preventing further movement of the tab element relative to the opening and dilatation of the cover.

Another aspect of the present disclosure, not corresponding to the wording of the claims, relates to a biological cover for a stent, which may comprise a biological material that may comprise a main body and at least one attachment portion protruding away from the main body. The main body may be adapted to be transformed into a (substantially) cylindrical configuration. This aspect may also be combined with features presented in connection with other aspects of the present disclosure.

For example, the biological cover may be produced by a method described herein.

The at least one attachment portion protruding away from the main body may be configured to provide mechanical fixation of the biological material to the stent. The at least one attachment portion protruding away from the main body may further be configured to form a loop-like element for mechanical fixation, with which a mechanical connection between the at least one attachment portion protruding away from the main body and the stent may be established.

The main body of the biological cover may be configured to be wound around an exterior of the stent (e.g., as a circumferential collar) and, by folding the at least one attachment portion protruding away from the main body into an opening of the stent, to be retained in the (substantially) cylindrical configuration.

It should be noted that both the main body and/or the at least one fastening portion protruding away from the main body can be provided in non-crosslinked form and in crosslinked form. In particular, the advantageous effects as described herein can be obtained thereby.

Such an embodiment of the biological cover may allow storage of the biological cover in a (substantially) two-dimensional form, wherein the biological cover can be converted to a (substantially) cylindrical configuration only upon use in clinical practice by attachment to a stent. This may contribute to simplified and space-saving storage of the biological material and allow modularization of biological covers relative to a stent.

Another aspect of the present invention relates to a device or apparatus for manufacturing a biological cover for a stent. The device comprises a counter element and a pressure generating means. The pressure element is adapted to be introduced into an inner lumen of a (substantially) cylindrical biological material to press the biological material onto the counter element.

The device is adapted to crosslink a biological cover for a stent, which may be provided in a (substantially) cylindrical configuration, in the presence of pressure and, if necessary, to crosslink regions of the biological material to be connected in the process.

The counter element may be formed in one or two parts or may comprise more than two parts. The counter element may be provided as a (two-part) outer mold defining, for example, a cylindrical opening which may be adapted to receive the biological material and optionally a preferably cylindrical stent therein. The mating element may be adapted to (fully) receive the same. The counter element may be configured to be dimensionally stable so as to withstand dilatation, for example, by a balloon catheter.

The apparatus may further comprise means for supplying and/or receiving a crosslinking agent. The pressure generating means may be provided, for example, as a dilatable tubing element (e.g., a balloon catheter).

It may further be possible to provide a stent that can be inserted into the biological material. In this case, the device may be configured to receive a stent and the (substantially) cylindrical biological material such that, crosslinking of the biological material can be performed on the stent such that a connection between the biological material and the stent can be enabled. This may include, for example, any of the further aspects discussed herein for connecting a stent and a cover. Thus, the stent and cover can be seamlessly connected.

Such a device can provide a simplified and economical manufacturing process for a cover for a stent, by joining two (overlapping) regions of the biological cover, which can be achieved in the presence of a crosslinking agent and pressure on the site or elements of the biological material to be joined. In particular, the crosslinking may be performed directly on the stent and optionally the crosslinking may simultaneously provide an (additional) connection to the stent.

For example, it may also be possible to enable a (final) biological cover in a clinical setting, which may enable adaptation of the biological cover by medical staff involved in the associated treatment of a patient. This may further enable targeted adaptation (e.g., in terms of customization) of the biological cover to the contemplated treatment. Another advantageous aspect of the device can be seen in the provision of a "ready-to-use" (biological) cover (e.g., pre-assembled), which can be designed to be storable (until its use, such as attachment to the stent).

The pressure generating means may be arranged to apply anisotropic pressure to the (substantially) cylindrically arranged biological material.

**In** some embodiments, the counter element may be provided to only partially surround the stent (e.g., in a cross-sectional view of the stent, only in a 180° angled portion). **In** this case, the counter element can be provided as a (polymeric) foam material that, when the biological material is pressed against the counter element, can apply a counter force.

To further stabilize and support the counterforce applied by the foam material, a rigid plate may be positioned below the foam material so that the rigid plate can act as a stabilizing foundation for the foam material.

The anisotropic pressure may be designed to be exerted (substantially) in one direction only (e.g., downward, i.e., facing a floor). Alternatively, it may be provided that the pressure is exerted such that it acts preferentially along a predetermined direction (e.g., the force component associated with the pressure may be more pronounced in a direction 5 times, 10 times, 15 times, etc. than along a direction transverse thereto).

The pressure generating means may be provided as a rod, preferably a rigid rod (e.g., a metal rod, a rod made of rigid plastic, etc.).

An anisotropic application of pressure to the (substantially) cylindrically shaped biological material may allow local, selective bonding of two (overlapping) regions of the to a (substantially) cylindrically shaped configuration without (unintentionally) cross-linking extensive regions of the biological material under pressure. This may enable a simplified and cost-effective manufacturing process of a biological cover for a stent.

In some examples, the device may be configured to perform the methods described herein to produce a biological cover for a stent.

It should also be noted that the cover is not limited to the use of biological materials, but that a non-biological material could also be used in principle. Such a non-biological material may include a plastic material (e.g., a PE material, etc.).

In general, it should also be noted that it is generally possible to start from prepared biological material/tissue in their native state, i.e. without chemical fixation with e.g. glutaraldehyde, which are stored in a moist state. In this case, however, it may be necessary to ensure, with increased effort, rapid and contamination-free processing of the native biological material, which must always be kept moist. In addition, a very high technical effort may be required to produce necessary molded parts from a moist native tissue, since internal mechanical stress during cutting may lead to unpredictable geometric distortions during cutting. Therefore, it is generally preferred to provide the biological material in a native dried state.

In collagen-containing tissue, a distinction is made between structural water, bound water and free water. Structural water, also called tightly bound water, is associated with stabilization of the triple helix structure. Intermolecular water molecules between triple helices and microfibrils are called bound water or contact water. Between the microfibrils and fibrils is free, unbound water. When water is removed, the collagen fibers lose their flexibility. If the removal of water is limited to free, unbound water, this process is reversible. However, if the drying process results in the removal of bound water molecules, the fiber network is irreversibly damaged. The absence of water causes a change in tissue conformation, which is accompanied by an increase in intra- and possibly also intermolecular hydrogen bonding. Complete rehydration of the fiber composite is thus precluded. The elastic properties of the collagen fibers are lost, and the tissue becomes brittle and fragile.

To enable structure-preserving drying of the tissue, i.e. stabilized drying, at least one hygroscopic exchange substance is used, which stabilizes the collagen structure in the event of water withdrawal. In this way, rehydration makes it possible to restore the initial state of the tissue. Suitable hygroscopic exchange materials include all biocompatible and non-volatile hygroscopic exchange materials, in particular hydrophilic hydrocarbons with a large number of hydroxyl groups, for example watersoluble sugar alcohols such as glycerol, or ethylene glycol or polyethylene glycol. A hygroscopic exchange substance can be, for example, glycerol or a mixture of two or more hygroscopic exchange substances, such as, for example, glycerol and polyethylene glycol (possibly with different molecular weights). The hygroscopic exchange substances can either be added individually, for example one after the other, or can be used as a mixture of the two substances, for example glycerol dissolved in polyethylene glycol. Preferred in the context of the invention is a sequence of glycerol in a first stabilization step, followed by a first PEG stabilization (e.g. with PEG 200), followed by a second PEG stabilization (e.g. with PEG 400). The second variant of two or more hygroscopic exchange materials also includes embodiments in which the same exchange material is used, such as polyethylene glycol, but in different versions, such as with different molecular weights. All of the above substances are characterized by their hygroscopic properties, low toxicity values and excellent water solubility. The ability to absorb and bind moisture from the environment opens up a wide range of applications for glycerin and polyethylene glycol (PEG) in various suitable mixing ratios to bring about the stabilization of collagen-containing tissue. In an exemplary stabilization step, the fabric is exposed to at least one solution containing glycerol and/or polyethylene glycol, wherein the fabric is exposed either to one of these solutions or to the two solutions successively in any order and composition as first and second solution or to both solutions simultaneously as a solution mixture - or also to a solution of glycerol and PEG in water. When drying tissue, e.g. for storage or transport of the tissue, the stabilization process is preferably carried out before drying. Optionally, decellularization of the collagen-containing tissue, e.g., using surfactin and deoxycholic acid, DNAse, alpha-galactosidase treatment, etc., can be performed to produce collagen-containing tissue with reduced immune response. The stabilization process may be carried out, for example, after decellularization by immersing the collagen-containing tissue in a series of one or more stabilizing solutions of glycerol and/or polyethylene glycol to sufficiently saturate the tissue with stabilizing agents, and ultimately to introduce a stable tissue into the ensuing drying process.

In general, the aspects described herein with respect to a cover for a stent can also be applied to other implants. The biological material can then be provided accordingly in a pattern suitable for the other implant in question.

The following figures serve to further illustrate aspects of the present invention and possible exemplary embodiments.
- Figs. 1A-1C: show a first exemplary embodiment of a biological cover for a stent;
- Figs. 2A-2C: show exemplary cut patterns of a biological material for producing a biological cover for a stent according to possible embodiments;
- Figs. 3A-3B: show an exemplary embodiment of a biological cover for a stent;
- Fig. 4: shows an exemplary embodiment of an attachment option of a biological cover to a stent;
- Figs. 5A-5C: show exemplary mechanical fasteners for a stent;
- Figs. 6A-6G: show exemplary manufacturing devices and processes for producing a biological cover for a stent;
- Figs. 7-9: show exemplary process diagrams for the production of a biological cover for a stent.

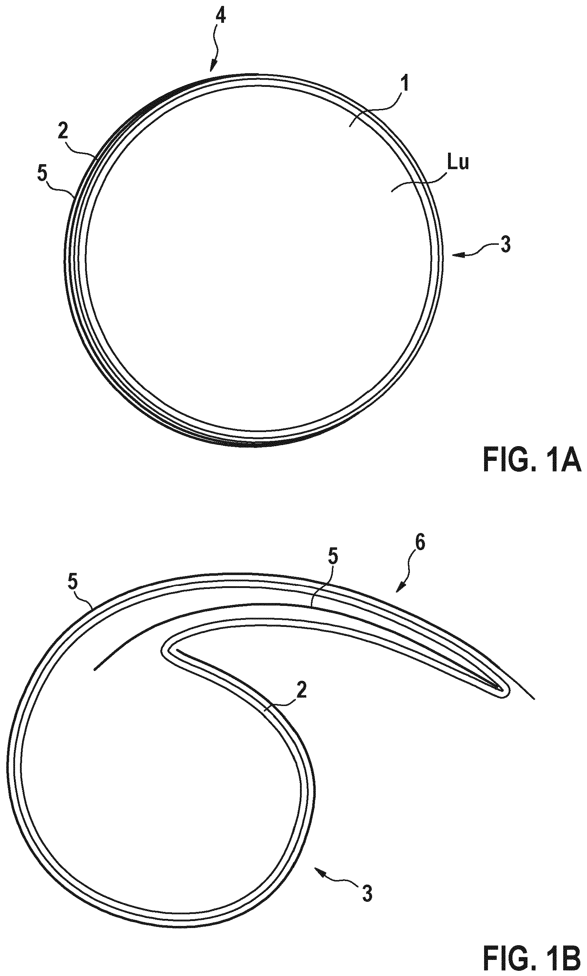

Figures 1A-1C show a cover for a stent according to a first embodiment.

Fig. 1A shows a cross-section of a stent 1, taken transversely to a longitudinal extension of the stent 1. Stent 1 may have an inner lumen Lu, which may be adapted to receive means to dilate the stent 1 in a radial direction and/or to be able to insert additional medical devices into the stent 1. Stent 1 preferably has a cylindrical shape or a circular cross-sectional shape. Stent 1 is further provided with a cover 3, preferably a biological cover 3, made of a first material 2 (e.g., a biological material such as a natively dried tissue (e.g., the use of natively dried pericardium may allow this cylindrical shape to already be formed from an originally flat, two-dimensional, native piece of tissue). Cover 3 is preferably attached to the stent 1 in such a way that it completely surrounds the stent 1, e.g. in a 360° geometry. Cover 3 may thereby be provided as described herein and is provided in a (substantially) cylindrical configuration so as to encase the stent 1.

Cover 3 comprises at least one overlapping region 4, which is configured as a fold, which may be obtained by folding at least a part of cover 3 along a longitudinal extension of stent 1, in itself. As a result of the folding, an overlapping region 4 is obtained, in which at least two layers of the first material may be superimposed.

In the sense of the solution according to the invention, it is also possible to implement the folding not with a single fold along the longitudinal axis of the stent 1. Thus, it is equally possible to technically implement several (smaller) folds along a circumferential direction. In this way, a symmetrical shaping of the stent 1 provided with a cover can be made possible.

Furthermore, a second material 5 is introduced into the overlapping area 4, which is selected in such a way that, when a crosslinking agent is added, the first material 2 is not bonded in the areas where the second material 5 separates two overlapping areas of the first material 2 from one another, and the overlapping areas of the first material 2 in question thus remain movable relative to one another. In this case, the second material 5 can merely be introduced into the overlapping region 4 during the cross-linking of the first material 2 and removed again after the cross-linking process has been completed. Alternatively, however, it may also be possible to leave the second material 5 in the overlapping area 5 beyond the crosslinking.

Fig. 1B shows the cover 3 of Fig. 1A in a cross-section (without the stent 1) in a partially unfolded state. In particular, at least part of the overlapping region created in the preparation step (Fig. 1A) may unfold, resulting in an at least partially unfolded region 6 of the cover 3. Fig. 1B further shows for better comprehensibility the second material 5, which (with reference to Fig. 1A) is introduced into the at least partially unfolded area 6. Additionally or alternatively, it is also possible to cover an outer side of the cover 3 (and more particularly of the first material 2) with the second material 5, so as to prevent unwanted cross-linking in the overlapping region 4. The second material 5 can also, for example, completely enclose the first material 2 in order to improve the crosslinking process.

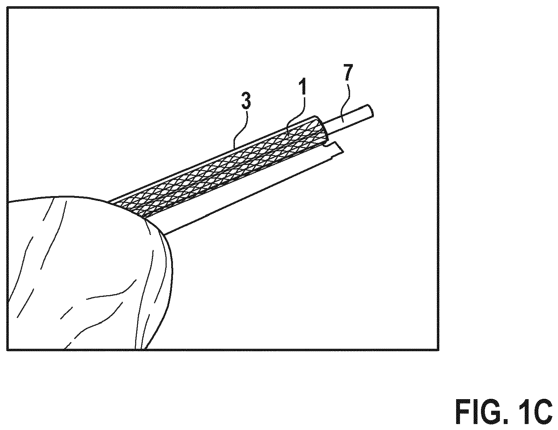

Fig. 1C shows the stent 1 in a prefabricated state, i.e. in a state in which it has been encased with the cover 3 and the overlapping region 4 has been provided with the second material 5. Furthermore, a rigid element 7 can be introduced into the inner lumen Lu of the stent 1. In this state of fabrication of the stent 1 a cross-linking takes place.

By means of this embodiment described with reference to Figures 1A-1C, it can be achieved during dilatation of the stent 1 that the cover 3 is subjected to only minimal mechanical stress, since as the diameter of the stent 1 increases, the cover 3 (and in particular the overlapping region 4) first unfolds before mechanical stress occurs in the plane of the first material 2. By dimensioning the first material 2, it can be made possible to avoid or selectively adjust the mechanical forces occurring during dilatation of the stent 1. The length of the overlapping zone 4 may depend on the desired initial and final diameter of the cover 3. For the stent 1 considered above with an exemplary diameter of 6 French in a crimped condition, the length of the overlapping zone may be about 4.7 mm in order to completely avoid mechanical stress in the plane of the first material 2.

In some applications, it may be possible to selectively omit the second material 5 at some locations so that the overlapping layers of the first material 2 resulting from folding can chemically bond (e.g., by crosslinking) in the overlapping region 5. For example, about 0.1% to 50% or 0.5% to 40% or 1% to 30% of the overlapping region may be bonded. A possible example of this may be a series of punctate bonding sites (e.g., punctate free sites spaced 1 mm, 2 mm, 3 mm, 4 mm, 5 mm, 10 mm, etc.), along the longitudinal axis of the stent 1 in the anterior (i.e., a distal) region of the folded first material 2. The point chemical bonds formed during chemical cross-linking under pressure can keep the fold in the overlapping region 4 on the stent 1, so that unwanted unfolding during implantation is safely avoided. Only when the stent 1 is dilated, e.g., with a balloon, can the created junctions become loose. In this way, the cover 3 (and thus the first material 2) can dilate together with the stent 1 into a cylindrical shape.

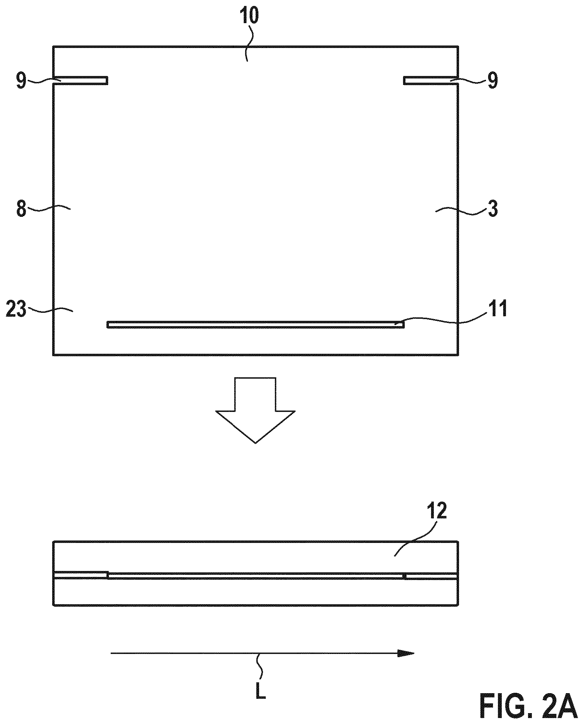

Figures 2A-2C show exemplary cut patterns for the cover 3 (and thus for the first material 2) according to one possible embodiment. It should be noted that aspects of this exemplary embodiment presented herein may also be combined with aspects of other embodiments.

Fig. 2A (top) shows a first exemplary cutting pattern (in an initially two-dimensional form) of a biological material for producing a cover 3 according to a possible embodiment. The cutting pattern may be provided with a (substantially) rectangular basic shape 8. The cutting pattern may further be produced from the biological material by means of a CO₂ laser, a pair of scissors, a scalpel, by punching, etc.

In addition, it is possible that at least two slits 9 are introduced into the (essentially) rectangular basic shape 8, which are introduced into the biological material on opposite sides of the rectangular basic shape 8, with respect to a longitudinal extension L of the rectangular basic shape 8. As a result of the insertion of the at least two slits 9, at least one tab element 10 can be formed in the rectangular basic shape 8. Thus, a tab element with a relatively narrow section can be created. The width of the slits can be adapted, for example, to the thickness of the material forming the cover 3.

Furthermore, at least one opening 11 can be worked into the biological material in the rectangular basic shape 8 on a side opposite the tab element. This opening 11 can thereby be worked into the biological material centered relative to a length of the longitudinal extension L. The opening 11 may further preferably be provided with a length corresponding to a lateral extent of the relatively narrow portion of the tab element, and a width which may correspond to the thickness of the material forming the cover 3.

Fig. 2A (bottom) shows the first exemplary cutaway pattern of Fig. 2A (top), wherein the at least one tab element 10 has been passed through the at least one opening 11, the biological material 23 (provided in the basic rectangular shape 8) thereby having been transferred to a (substantially) cylindrical configuration 12 and in this form could be used as a cover 3 for the stent 1. The cover 3 thus obtained can thereby keep blood away from an outer wall of the stent 1 and thus contribute to increased biocompatibility or hemocompatibility of the stent 1.

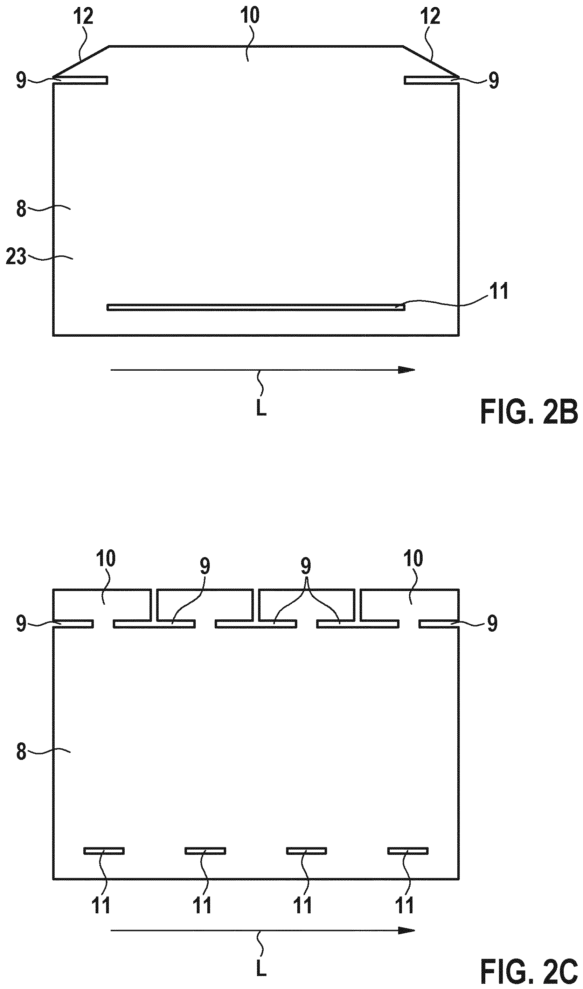

Fig. 2B shows another exemplary cutting pattern for the biological material for producing a cover 3 for the stent 1 according to a further embodiment. In this possible embodiment, the biological material may be provided as the biological material already described with reference to Fig. 2A (above). In addition, the tab element 10, at its opposite ends (with respect to the longitudinal extension L), may be provided with flattened edges 12. Such a provision of the tab element 10 can thereby support a simplified passage of the tab element 10 through the opening 11.

Fig. 2C shows another exemplary cutting pattern for the biological material for producing a cover 3 for the stent 1 according to a further embodiment. In this possible embodiment, the at least one tab element 10 (as has been described with reference to Fig. 2A (above)) is subdivided into a number of tab elements 10 by making further slits 9. Generally, two, three, four or more tab elements 10 could be provided. Similarly, a corresponding number of openings 11 have been provided. Preferably, each tab element 10 can be assigned an opening 11. Such a cutting pattern may thereby allow a more flexible cover 3 for the stent 1.

It should be further noted, with regard to the aforementioned exemplary cutting patterns, that stabilizers incorporated into the preferably natively dried biological material, such as glycerol, PEG200, PEG400, can, due to their high viscosity and the formation of hydrogen bonds, bring about a mechanically stable connection necessary for manual handling and also processing in and with technical devices. However, this connection can still be reversibly detachable, so that exact positioning or repositioning of the still native tissue can still be made possible (e.g. of tab element 10 relative to opening 11).

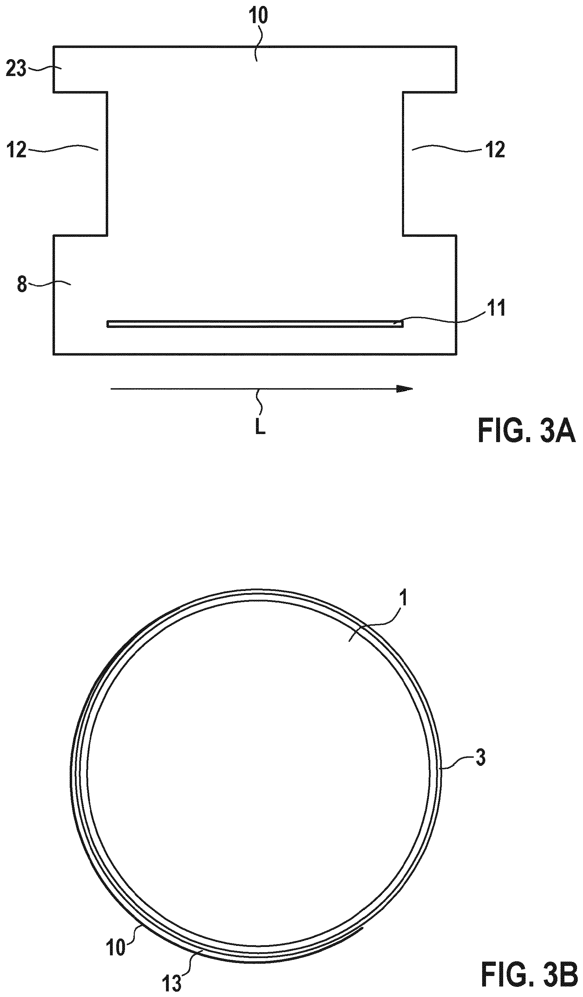

Figures 3A and 3B illustrate another exemplary embodiment of a shroud 3. It should be noted that aspects of this embodiment may be combined with aspects of other embodiments mentioned herein.

Fig. 3A shows an example of a possible cutting pattern for the biological material in a (substantially) rectangular base shape 8, where the rectangular base shape 8 and the biological material can be provided as described with reference to Figures 2A-2C.

In addition, two elements 12 can be cut out of the rectangular basic shape 8, on two opposite sides (transverse to the longitudinal extension L). The two elements 12 may preferably have a rectangular shape, but are not limited thereto. Thus, in some embodiments, it may be preferred to recess the elements 12 in a hyperbola-like shape, etc. By recessing the two elements 12, a tab element 10 can be formed on one side of the biological material. The relatively narrow portion of the tab element 10 can thus be formed to be significantly longer than the thickness of the biological material, e.g., at least ten times longer, at least 100 times longer, at least 500 times longer.

Further, the biological material may be provided with an opening 11 in the rectangular base shape 8. As described with reference to Figures 2A-2C, the combination of the tab element 10 and the opening 11 may allow the two-dimensional basic shape to be converted into a (substantially) cylindrical configuration, which may be achieved by passing the tab element 10 through the opening 11. This embodiment may allow the tab element 10 not to remain rigidly in the opening 11, but to allow the tab element 10 to slide along its direction of passage through the opening 11 by means of the recessed elements 12. This can be particularly advantageous in the case of dilatable stents, since in this case the cover 3 can follow a dilatation (and thus an increase in a diameter of a dilated stent 1) by sliding of the tab element 10 relative to the opening 11 occurrence of a mechanical tension force on the biological material (whereby elongation at rupture can be prevented).

For this embodiment, biological material stored in a moist state can also be used initially for the production of the cutting pattern, which is only cut into the required geometric shape (i.e. the exemplary cutting pattern) after crosslinking in e.g. glutaraldehyde.

Fig. 3B shows the cover 3 as described with reference to Fig. 3A after it has been attached to the outside of the stent 1.

The slipperiness of the tab element 10 relative to the opening 11 may thus result in an overlapping region 13, which may increase from a first cylinder radius to a second cylinder radius as the stent 1 expands, thus following the increase in cylinder radius. An advantageous effect of this mechanism can be seen in the fact that the cover 3 always surrounds the stent 1 in a closed form.

The biological material may further be attached to the stent 1 in a region of the opening 11 (e.g., by cross-linking and/or mechanical fasteners, etc.). The cover 3 attached to the stent 1 is subjected to cross-linking. For this purpose, it is advantageous to introduce a second material (not shown) into the overlapping area 13, between two superimposed layers of biological material (as described with reference to Figures 1A-1C) to prevent crosslinking of the superimposed layers of biological material, at least locally. After the crosslinking step, the second material can be removed from the overlapping region 13. The crosslinking can be followed by a stabilizing drying.

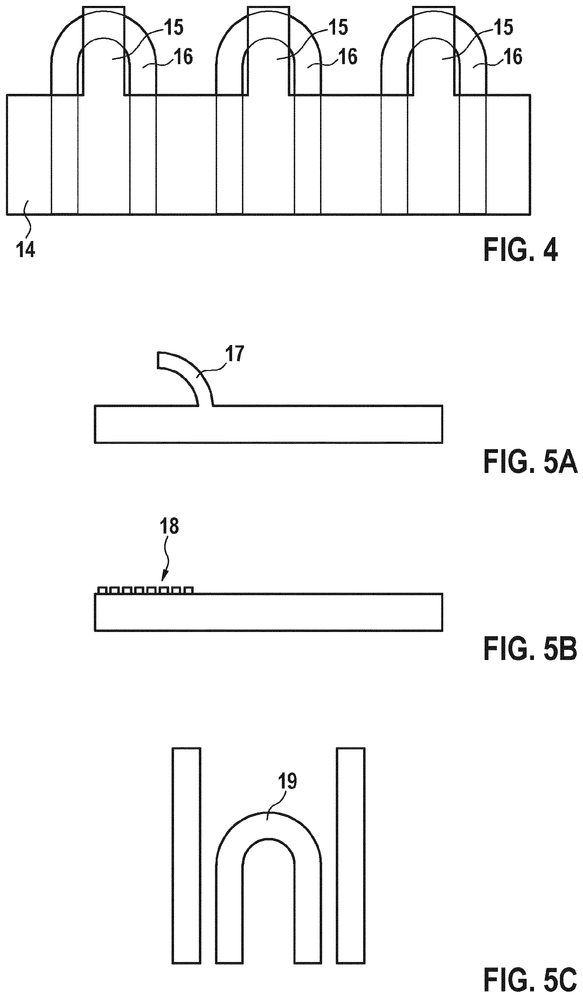

Fig. 4 shows an exemplary cutaway pattern for the biological material according to another possible embodiment for making a cover 3 for the stent 1. It should be noted that aspects of this embodiment may be combined with aspects of other embodiments mentioned herein. The biological material can be provided in a (substantially) rectangular basic shape as the main body 14. In addition, the biological material can be provided with at least one attachment section 15 protruding away from the main body 14.

By means of the at least one attachment portion 15 protruding away from the main body 14, it can be made possible to cover the stent 1 by means of the main body 14, while the attachment portion 15 protruding away from the main body 14 can be used to guide the biological material through an opening on the stent 1 (e.g. formed by an element 16 of its wire mesh) and thus form a tab-like attachment element (e.g. as a loop), whereby the biological material can be attached to the stent 1. In particular, the formation of the (fixed) loops can thereby be achieved by a crosslinking process (preferably under pressure).

It is also possible that biological material is completely folded over inward at the edge of the stent 1, which then forms a circumferential collar at the edge of the stent 1. The collar can be formed on only one or both sides of the stent 1. Crosslinking under pressure can achieve a form fit of the biological material around the stent 1 at the edge.

Figures 5A-5C show exemplary modifications to the exterior of the stent 1 according to another embodiment. It should be noted that aspects of this embodiment may be combined with aspects of other embodiments mentioned herein.

Fig. 5A shows an example of an outer surface of the stent 1, which is provided with a hook or hook-like structure 17 as a mechanical fastening element to attach the biological material to it as a cover 3. The biological material can be attached to the hook 17 by piercing it. The hook 17 may be attached not only to an end portion (e.g., a proximal end and/or a distal end) of the stent 1, but also along the longitudinal extension L. The hook 7 may optionally be bent over toward the stent with a tool after the attachment of the biological material, thereby further preventing accidental slippage of the biological material from the stent 1.

Fig. 5B shows an example of a further possible design of the outer side of the stent 1, which can be used as an alternative or in addition to the modification of the outer side of the stent 1 described with reference to Fig. 5A. In particular, Fig. 5B thereby shows the outer side of the stent 1, wherein the outer side has been provided by means of a roughened surface element 18, as a mechanical fastening element. For example, the biological material can be pressed into the surface element during crosslinking, so that a connection is made.

These modifications, as described with reference to Figures 5A and 5B, of the stent 1 may be used for sole or additional mechanical fixation or attachment of the biological material to the stent 1 and may be designed independently of the initial state of the biological material.

Fig. 5C shows another exemplary mechanical fastening element, which can be used alone to fasten the biological material to the stent 1 or, for example, in combination with the mechanical fastening elements described with reference to Figs. 5A and/or 5B. In this regard, Fig. 5C shows clamp element 19, which can preferably be hooked to one or both ends of the stent (regardless of the processing of the biological material) to further secure the biological element to the stent 1. This may help to ensure adequate retention of the biological material to the stent 1 during implantation of the stent 1.

On the one hand, the additional modifications serve to provide additional mechanical fixation of a biological material processed on the stent 1. On the other hand, independently processed biological materials can also be fixed to the stent 1 (e.g. by pressing the biological material against the mechanical fixation elements (see below for a detailed description of this pressing). The mechanical fixation may allow the biological material to be fixed during storage of the ready-built encased stent and the encasement 3 to be held securely on the stent 1 during catheter-based implantation.

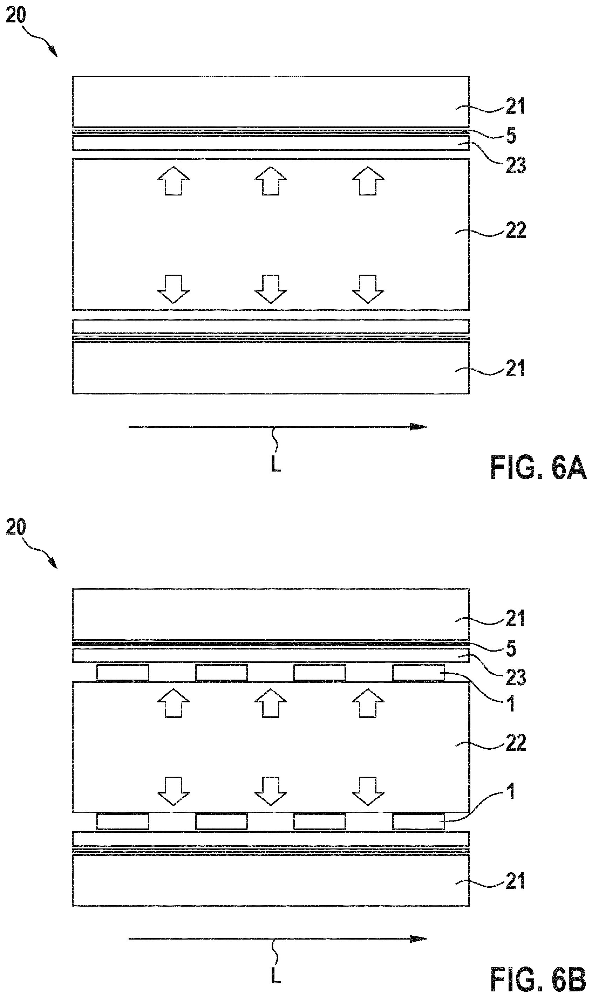

Figures 6A-6G show exemplary embodiments of a device for producing a biological cover for a stent.

Fig. 6A shows a first possible embodiment for a device 20 for manufacturing a biological coating 3 in a cross-sectional view shown along a longitudinal extension. The device 20 comprises an e.g. one- or two-part counter element 21, which is preferably provided with a cylindrical receiving means for receiving therein the preferably cylindrically configured pressure generating means 22.

Optionally, it is possible, e.g. for longer lengths of the stent 1, to provide the counter element 21 with holes, to design the inner surface of the counter element 21 with fine superficial channels or with inherent porosity (e.g. ceramic). This may allow additional convective and diffusive mass transfer.

The mating element 21 may also have surface structures that extend into open regions of the stent 1 (Fig. 6B). This may allow geometric adaptation of the biological material 23 into the stent 1, providing additional support of the biological material 23 beyond mere embedding of the stent (esp. the stent strut) into the planar biological material 23.

The pressure generating means 22 (e.g., a hydraulically and/or pneumatically dilatable balloon catheter) may be provided to dilate along a radial direction (perpendicular to the longitudinal direction L). A biological material 23 to be crosslinked may further be provided around the pressure generating means 22, which may preferably be provided as a (substantially) cylindrical arrangement and may be pressed against the counter elements 21 by the dilatation of the pressure generating means 22 (exemplarily represented by the direction of the arrows shown in Fig. 6A).

A layer of a second material 5, which may be provided as described herein (e.g., with reference to Figures 1A- 1C), may be interposed between the biological material 23 as well as the counter elements 21. In this arrangement, cross-linking of the biological material 23 can be achieved.

Fig. 6B shows a second possible embodiment for a device 20 for manufacturing a biological coating 3 in a cross-sectional view shown along a longitudinal extension. The manufacturing device shown in reference to Fig. 6B is thereby based on the manufacturing device shown in reference to Fig. 6A, whereby a stent 1 can additionally be introduced between the pressure-generating means 22 and the biological material 23 (preferably from natively dried pericardium) and thus it can be made possible to also carry out a cross-linking of the biological material 23 onto the stent 1.

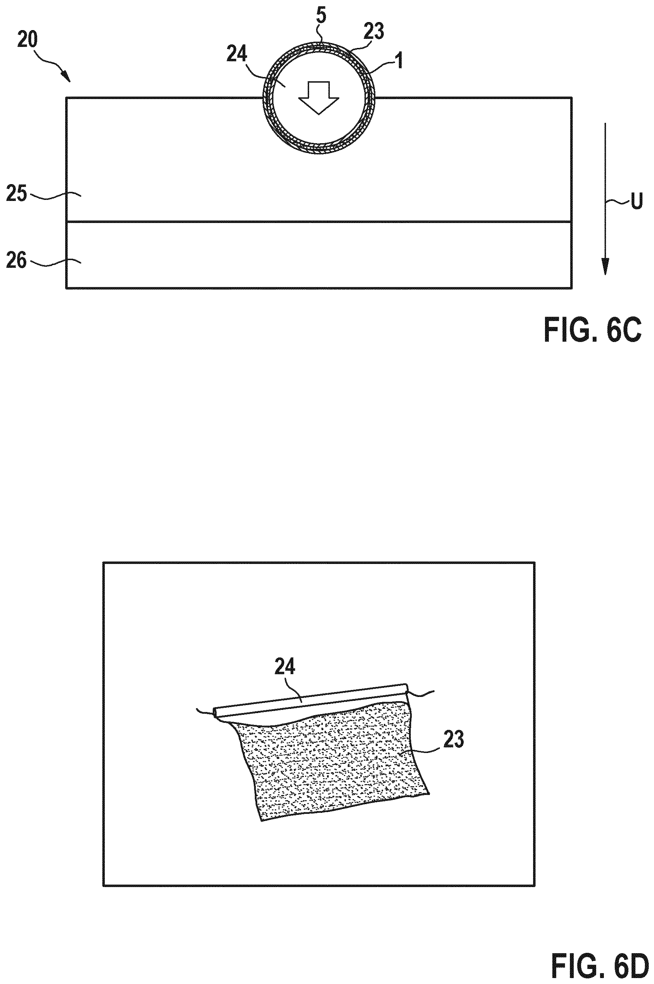

Fig 6C shows a third possible embodiment for a device 20 for manufacturing a biological casing 3 in a cross-sectional view of the device, perpendicular to its longitudinal extension. Instead of the pressure generating means 22 shown in Figs. 6A and 6B, a pin-like element 24 (e.g., a steel pin) may be introduced into the inner lumen of the stent 1 with respect to the device as shown in Fig. 6C.

By exerting pressure by means of the pin-like element 24 in the direction U, the cylindrical arrangement (which can be designed as in Fig. 6A or 6B) can be pressed into a foam element 25 (e.g. polyurethane with a compression hardness of e.g. 60 kPa). The biological material 23 is preferably arranged in such a way that the parts to be crosslinked are at the bottom, in the U direction.

The foam member 25 may be provided such that it applies a counterforce against the application of pressure, such that an application of pressure to the areas of the biological material 23 to be crosslinked may be achieved. The foam element 25 may further be mounted on a (rigid) plate 26 so as to provide a stable base for the application of pressure by the pin-like element 24.

It is further noted that device 20 according to Fig. 6C can also be used without stent 1.

Furthermore, it is also possible to additionally insert the second material 5 between (overlapping) layers of the biological material 23, which are not to be crosslinked with each other.

In this context, it can be further noted that the elongation at break of pericardium crosslinked under tension in the plane of the biological material 23 or under external pressure is lower than the elongation at break of pericardium crosslinked freely under minimal external force. In this context, free cross-linking of the pericardium can be motivated without external pressure or internal mechanical tension on the collagen fibers during cross-linking. External pressure can thereby be applied only at the point where a stable connection of parts of the biological material 23 is required.

Using the fabrication device 20 as shown in Fig. 6C, the technical arrangement allows a high mechanical pressure to be applied to the junction of the biological material 23 for tissue bonding, while in most of the biological cover 3, crosslinking occurs with no or much lower mechanical pressure. A biological cover 3 with an overall greater elongation at break can thus be obtained.

The previously discussed manufacturing devices may further have in common that during crosslinking under pressure, a sufficient exchange of fluids may be ensured, which may be supported in particular by the second material 5. This may be aided, on the one hand, by the removal of water from the biological material 23 during compression by the application of pressure and, on the other hand, by the supply of the crosslinking agent (e.g., a glutaraldehyde fixative solution) to the biological material 23. Preferably, the second material 5 can be provided as a polyester screen mesh with 100-180 threads per cm. The mesh size of the fabric may range from 10-60µm, and a thickness of the biological material 23 may be preferred to be comparatively thin, with a thickness of about 40µm-70µm being advantageous.

In any of the aforementioned embodiments for the manufacturing device, DPBS (Dulbecco's Phosphate Buffered Saline Solution; pH 7.4) may be introduced into the device in a state of pressurization of the biological material 23. Under pressure, stabilizers stored via the drainage can be replaced with DPBS, thus rehydrating biological material 23. The built-up pressure can also compress and reduce the thickness of the biological material 23. At the junctions of the biological material 23, the application of pressure can achieve a mechanically intimate connection between the portions of the biological material 23 by locally displacing the collagen fibers. The process of rehydration can optionally be assisted by an alternating pressure profile, which can assist the mass transfer by a pumping motion of the internal dilatable structure.

After this preconditioning, glutaraldehyde can be added to the DPBS to form, for example, an exemplary concentration of 0.1%-1.5%, preferably 0.5% glutaraldehyde in DPBS. The glutaraldehyde may cause the biological material 23 to be chemically internally crosslinked, and may also cause chemical bonds to be formed at the junctions of the biological material 23.

The crosslinking can be carried out under static pressure or preferably an alternating pressure profile, which can support the mass transfer by a pumping movement of the internal dilatable structure. **In** the applicant's experiments, crosslinking under pressure for 2 days at 37°C or for 4 days at room temperature, for example, has proved successful, although parameter constellations deviating from this can also provide a satisfactory result (such as crosslinking in a temperature range of 15-45°C over a period of 1-5 days).

The goal of crosslinking in the mold under radial pressure may be to achieve a dimensionally stable design of the desired three-dimensional geometry of the biological material 23, to reduce the material thickness of the biological material 23 to typically 30-70 micrometers, and to provide a mechanically stable bond between contacting layers of the biological material 23.

Once this state has been reached, stent 1 with applied biological coating 3 can be removed from the device 20 and optionally completely crosslinked in further crosslinking steps outside the device 20 while constantly changing the crosslinking agent. By this additional post-fixation, the amount of not yet fixed free amino groups can be reduced with optimal accessibility of the crosslinking agent to the biological material 23. Thus, a final crosslinking state comparable to standard processed pericardial patches can be achieved. Further optional process steps, such as final crosslinking at elevated temperature or *capping* of unsaturated aldehyde groups, may also be possible in order to achieve improved properties of the biological material 23 with regard to *in vivo* calcification.

Figures 6D-6E show the manufacturing process of the biological coating 3 in connection with the manufacturing device as described with reference to Fig. 6C.

Here, Fig. 6D shows the biological material 23 in two-dimensional form, which is at least partially attached to the pin-like element 24.

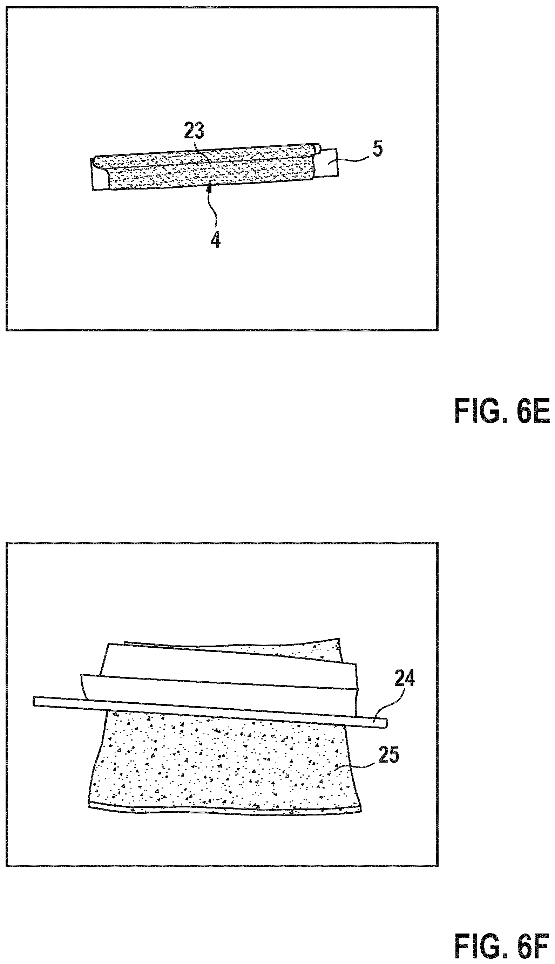

Fig. 6E shows a subsequent processing step of the biological material 23, which is attached to the pin-like element 24 forming at least one overlapping area 4. The overlapping area 4 is provided, between the two overlapping layers of biological material 23, with the second material 5.

Fig. 6F shows exemplarily the pin-like element 24 when this has been encased by the biological material 23 and further a second material 5 is introduced into two overlapping layers of the biological element 23, in the overlapping area 4. Further, in Fig. 6F, the arrangement thus obtained is superimposed on the foam element 25.

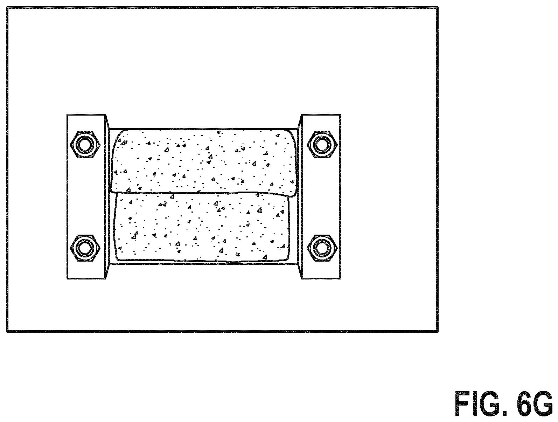

Fig. 6G shows the manufacturing device of Fig. 6F exemplarily in a closed arrangement, in which a predefined pressure generation on the areas of the biological material 23 to be crosslinked can be enabled by pressing the element 24 in a defined manner.

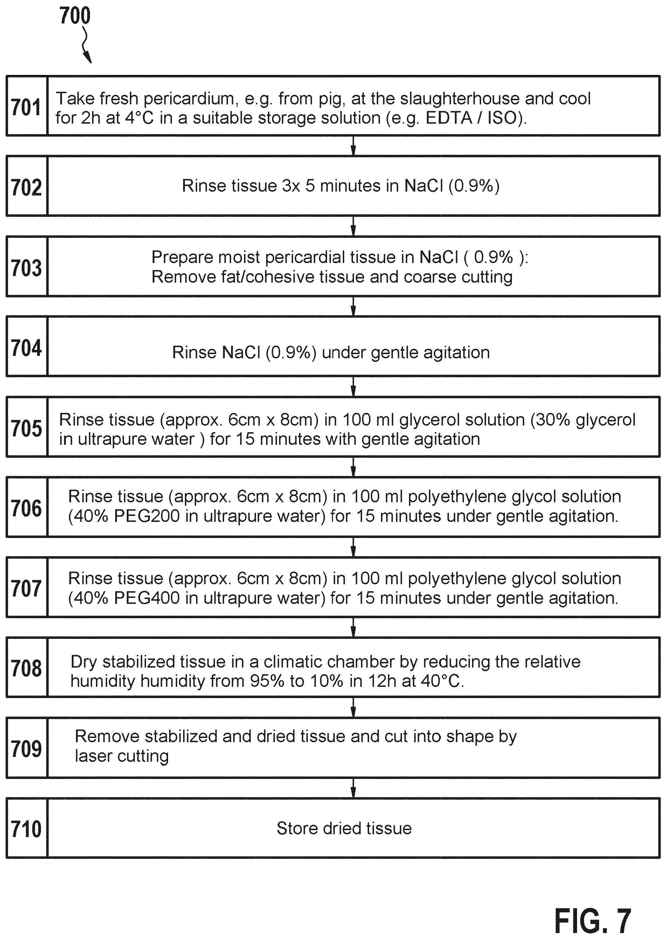

Fig. 7 shows an exemplary process diagram for the production of natively dried pericardium as starting material for the production of biological covers 3 for a stent 1. The manufacturing process 700 may exemplarily start with step 701, in which step a pericardial tissue is selected as the biological material 23. This can come directly from a pig, which is obtained as part of the slaughter process and can be stored at a sufficiently low temperature of 1-6°C, preferably at 4°C for a period of 1h - 4h, preferably for 2h, in a storage solution (e.g. EDTA which is ethylene diamine tetraacetic acid )/ISO, etc.) until further processing. As a subsequent step (step 702), it is possible to rinse the biological material several times (e.g., twice, three times, four times, five times, etc.), preferably three times, in a saline solution (with a concentration of 0.5%-1.5%, preferably 0.9%). Each rinsing process can take e.g. 3 minutes, 4 minutes, 5 minutes, 6 minutes, 7 minutes, etc., but preferably 3 minutes. Any value between these values may also be possible within aspects of the present invention. In step 703, the biological tissue 23 may be prepared (e.g., wet in NaCl at a concentration of, e.g., 0.9%) for subsequent process steps. This may include removal of fatty and/or connective tissue and rough cutting of the biological material 23 into the required shape. In step 704, a new rinsing of the biological material 23 in NaCl (with a preferred concentration of 0.9% can be carried out) with slight agitation. It should be noted that the rinsing process is not limited to NaCl and other suitable rinsing solutions may also be used. This may be followed, in step 705, by a rinsing process of the biological material, which has preferably been cut into a basic rectangular shape (e.g., 6 cm x 8 cm), in preferably 100 ml of glycerol solution (20-40% glycerol (preferably 30%) in ultrapure water) with gentle agitation for 5-20 minutes, preferably for 15 minutes.

In step 706, a further rinsing process of the biological material 23 in 100 ml polyethylene glycol solution (10-50%, preferably 40%, PEG200 in ultrapure water) may follow. The rinsing time, under gentle agitation may be 10-25 minutes, preferably 15 minutes. In step 707, another rinsing step can follow, in which the biological material can be rinsed in 100 ml of polyethylene glycol solution (10-50%, preferably 40%, PEG400 in ultrapure water). The rinsing time, under gentle agitation can be 10-25 minutes, preferably 15 minutes. In step 708, the biological material 23 can be dried in a climate chamber by reducing the relative humidity from, for example, 90-98%, preferably 95%, to 5-15%, preferably 10%, in 5-15 hours, preferably 12h, at 30-50°C, preferably at 40°C. In step 709, the biological material 23 thus stabilized and dried can be removed and laser cut into the desired shape. In step 710, the dried fabric can be further stored until it is used.

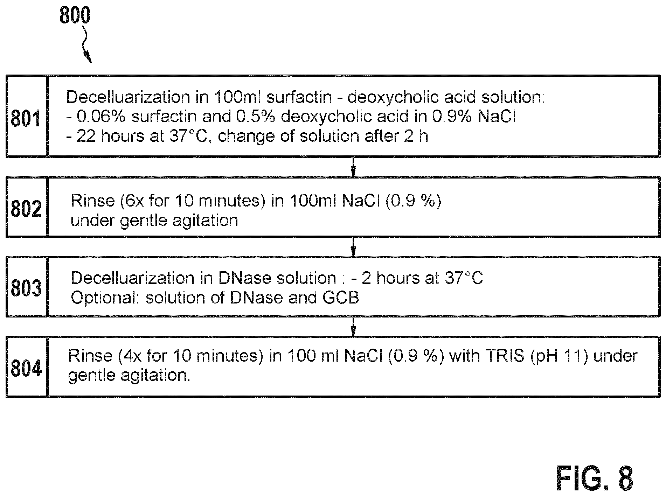

Fig. 8 shows an exemplary process diagram, for methods 800 for producing natively dried pericardium, which can be carried out between process steps 704 and 705 (as described with reference to Fig. 7). Step 801 comprises decellularizing the biological material 23 in 100ml in, for example, surfactin-deoxycholic acid solution, for example comprising 0.06% surfactin and 0.5% deoxycholic acid in 0.9% NaCl. This process can be performed over a period of 15-30 hours, preferably for 22 hours at a temperature of 30-40°C, preferably at 37°C. Furthermore, the solution can be changed after 1 h, 2 h, 3 h, 4 h, etc., preferably after 2 h. In step 802, rinsing of the biological material 23 in 100 ml of NaCl (e.g., 0.5-1.5%, preferably 0.9%) can be performed with gentle agitation. The rinsing process may be repeated for 2-10 times (preferably six times), and each rinsing process may be performed for 5-15 minutes, preferably 10 minutes. In step 803, decellularization in DNase solution may follow. This can be done for 1-3 hours (preferably 2 hours) at 30-40°C, preferably at 37°C. Optionally, step 803 can be performed in the presence of a solution of DNase and GCB.

In step 804, rinsing of biological material 23 in 100 ml of NaCl (e.g., 0.5-1.5%, preferably 0.9%) can be performed with gentle agitation. Additionally, TRIS (e.g., at pH 11) may be added to the NaCl solution. Step 805 may be followed by rinsing the biological material 23 for one to five times (preferably once) for 5-20 minutes (preferably 15 minutes) in 100 ml of ethanol (60-80%, preferably 70%) at 30-40°C, preferably at 37°C.

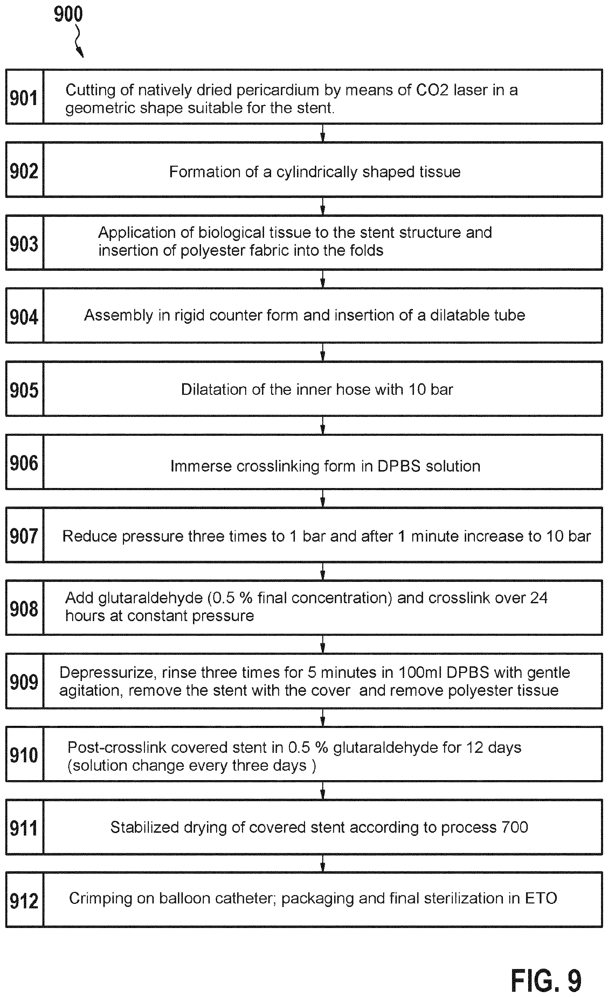

Fig. 9 shows another exemplary process diagram, for process 900, for producing a biological cover 3 for stent 1 by crosslinking under pressure. In step 901, native dried pericardium can be cut into a geometric shape suitable for the stent 1 using a CO2 laser. In step 902, forming a (substantially) cylindrical arrangement of the biological material 23 is performed. In step 903, arranging biological material 23 and insertion of the second material 5(e.g., polyester fabric) in the overlapping areas 4(folds of the biological material 23) is performed. In step 904, assembly into the rigid outer mold (e.g., counter element 21) may occur. Further, the pressurizing means 22 (e.g., a dilatable tube) is inserted into the biological material 23. In step 905, the pressurizing agent can be dilated, e.g., by applying a pressure of 5-15 bar, preferably 10 bar. In step 906, the crosslinking form thus constructed can be placed in DPBS solution. In step 907, the pressure can be reduced to 0.5-2 bar, preferably 1 bar, and after 1-5 minutes, preferably after 1 minute, it can be increased again to e.g. 10 bar. In step 908, glutaraldehyde (at a concentration of 0.2-1%, preferably 0.5%) can be added and the biological material 23 can thus be crosslinked (at preferably constant pressure) for a period of 20-30 hours, preferably for 24 hours. In step 909, the pressure can be released and the biological material 23, rinsed three to five times, preferably three times, for 1-10 minutes, preferably for 5 minutes, in 100ml DPBS with gentle agitation. Stent 1 can then be removed with the biological cover 3 applied and the second material 5 (e.g., the polyester fabric) removed. In step 910, the covered *stent* thus obtained can be freely post-crosslinked in 0.1-1%, preferably 0.5%, glutaraldehyde for 12 days, preferably changing the solution used every three days. According to step 911, drying of the encased stent 1 may subsequently be performed at least partially according to process 700. In step 912, crimping of the produced biological cover 3 onto a balloon catheter can be performed. The product thus obtained can be packaged and finally sterilized in e.g. ETO (ethylene oxide).

Furthermore, it should be noted in general that collagen-containing biological materials 23 such as pericardium can exhibit a fundamentally very similar mechanical behavior due to the microstructural composition consisting of an irregular network of collagen fibrils. When the biological material is stretched in the plane, the mechanical stress can initially increase very slowly with the stretch, since the collagen fibers can initially be aligned along the direction of the force. After alignment of the fibers, the collagen fibers can support mechanical load and a linear-elastic increase in mechanical stress with strain can occur. Above a certain mechanical stress, failure of progressively more collagen fibrils occurs until complete mechanical failure of the material. The associated elongation and stress at break may depend on a variety of possible initial parameters (e.g. tissue type, thickness, homogeneity and internal fiber distribution in the native starting material, etc.) and on a variety of process parameters (e.g. mechanical load during preparation, crosslinking under mechanical tensile load, etc.). Collagen-containing tissues processed in this way typically exhibit elongations at break of 5-15%, with a maximum of about 30%.

For the encasement of an encased stent 1, it must be ensured that no mechanical failure of the biological encasement 3 due to the formation of substantial holes occurs during implantation and use of the stent 1. Given elongations at rupture of the biological material 23 up to a maximum of about 30%, this may not be guaranteed or possible for typical balloon-expandable coronary stents. For example, the cover of a covered stent in current clinical practice with a cover 3 made of a polymer can be stretched by about 250% when dilated from 6 French in the crimped state to 5 mm in the dilated state. Consequently, the required elongation at break may be about an order of magnitude greater than the elongation at break of typical collagen-containing biological materials, such as pericardium.

With regard to the crosslinking agent used, it can be stated that this can be an aldehyde-containing solution or selected from the group consisting of glutaraldehyde, carbodiimide, formaldehyde, glutaraldehyde acetals, acyl azides, cyanimide, genipin, tannin, pentagalloyl glucose, phytate, proanthocyanidin, reuterin and/or epoxy compounds. The crosslinking agent may be a glutaraldehyde or a glutaraldehyde solution, preferably a glutaraldehyde solution having a concentration between 0 and 2% by volume of glutaraldehyde, more preferably between 0.01 and 1% by volume of glutaraldehyde, most preferably 0.5% by volume. Glutaraldehyde.

The second material 5 (which may act as a drainage agent, among other things) may be a (permeable) organic polymer layer, preferably a (permeable) polyester layer or a polyester nonwoven. The second material 5 may have a mesh size or pore size that allows the crosslinking solution and/or water to pass through the pores or meshes of the second material 5. This allows sufficient contact of the biological material 23 with the crosslinking solution and/or removal of the crosslinking solution and/or water away from the biological material 23. The second material 5 may have a mesh size or pore size of less than 60 µm, preferably in the range of 10 µm to 60 µm. Mesh sizes or pore sizes of less than 60 µm result in uniform tissue surfaces. Mesh sizes or pore sizes greater than 60 µm result in uneven tissue surfaces, such that an imprinted structure may be visible to the naked eye on the surface of the biological material 23. However, if desired, mesh sizes or pore sizes larger than 60 µm may be used to imprint structures (raised areas and/or depressions) on the surface of the biological material 23 or to provide a roughening of the tissue surface. The second material 5 may have a thickness of 40 µm to 70 µm.

## Claims

1. Method for producing a biological cover for a stent (3) comprising:
providing a first material (2) having a main body (14) in a cylindrical configuration and having at least one fold in the first material and having a second material (5) arranged within the at least one fold, wherein the first material (2) is a biological material (23);
pressing the first material (2) and the second material (5) onto a counter member (21) being arranged outside the cylindrical configuration; and
application of a crosslinking agent to the first material (2), **characterized in that** the first material (2) comprises crosslinkable groups which can be crosslinked by use of the crosslinking agent and wherein the second material (2) is a material which cannot be crosslinked to the first material (2) by use of the crosslinking agent.

2. The method according to claim 1, wherein the first material (2) comprises several folds.

3. The method according to claim 1 or 2, wherein the second material (2) is made of an organic polymer.

4. The method of any of the preceding claims, wherein the first material (2) further comprises at least one tab element (10) and at least one opening (11) for the respective tab element (10) to pass through.

5. The method of claim 4, wherein the at least one opening (11) is a slot.

6. The method of claim 4 or 5, wherein the cylindrical configuration is obtained by bending the first material (2) into the cylindrical configuration and fixing the cylindrical configuration by passing the least one tab element (10) through the respective at least one opening (11) to form a tab-and-slot connection.

7. The method of any one of claims 4 to 6, wherein the at least one tab element (10) comprises a middle portion being connected to a head portion, and wherein the middle portion has a smaller size than the head portion and the size of the middle portion is smaller or equal than the size of the respective at least one opening (11).

8. The method of any of the preceding claims, wherein the first material (2) further comprises at least one attachment portion (15) protruding from the main body (14).

9. The method according to claim 8, wherein the at least one attachment portion (15) is attached to a stent (1), preferably to at least one mechanical fastening element (17, 18, 19) of the stent.

10. A biological cover (3) for a stent made of a biological material (23), preferably obtained by a method according to any one of the preceding claims, comprising a main body (14) of the biological material in a cylindrical configuration having a first cylinder diameter; and at least one fold in the main body, wherein the at least one fold is configured to at least partially unfold upon radial dilation of the biological material to permit enlargement of the cylindrical configuration to a second cylinder diameter, **characterized in that** the biological material is crosslinked.

11. The biological cover (3) of claim 10, further comprising at least one tab element (10) and at least one opening (11) for the respective tab element (10) to pass through, and wherein the at least one tab element (10) is passed through the respective at least one opening (11) to form a tab-and-slot connection.

12. The biological cover (3) of claim 11, wherein the tab element (10) is movable relative to the opening along a direction of passing through.

13. The biological cover (3) of any one of the claims 10 to 12, wherein the biological material (23) further comprises at least one attachment portion (15) protruding from the main body (14) and which is attachable to a stent.

14. The biological cover (3) of any one of the claims 10 to 13, wherein the biological material (23) is a collagen-containing tissue, preferably pericardial tissue.

15. A device (20) for producing a biological cover (3) according to the method of any one of the claims 1 to 9, comprising a counter element (21) made of a polymeric foam, a pressure generating means (22) adapted to be introduced into an inner lumen (Lu) of the cylindrical biological material (23) to press the biological material (23) onto the counter element (21), wherein the pressure generating means (24) is adapted to apply anisotropic pressure to the cylindrical biological material (23).

## Patentansprüche

1. Verfahren zur Herstellung einer biologischen Ummantelung für einen Stent (3), umfassend:
Bereitstellen eines ersten Materials (2) mit einem Hauptkörper (14) in einer zylindrischen Konfiguration und mit mindestens einer Falte in dem ersten Material und mit einem zweiten Material (5), das innerhalb der mindestens einen Falte angeordnet ist, wobei das erste Material (2) ein biologisches Material (23) ist;
Pressen des ersten Materials (2) und des zweiten Materials (5) auf ein Gegenelement (21), das außerhalb der zylindrischen Konfiguration angeordnet ist; und
Aufbringen eines Vernetzungsmittels auf das erste Material (2), **dadurch gekennzeichnet, dass** das erste Material (2) vernetzbare Gruppen umfasst, die durch Verwendung des Vernetzungsmittels vernetzt werden können, und wobei das zweite Material (2) ein Material ist, das durch Verwendung des Vernetzungsmittels nicht mit dem ersten Material (2) vernetzt werden kann.

2. Verfahren nach Anspruch 1, wobei das erste Material (2) mehrere Falten aufweist.

3. Verfahren nach Anspruch 1 oder 2, wobei das zweite Material (2) aus einem organischen Polymer hergestellt ist.

4. Verfahren nach einem der vorhergehenden Ansprüche, wobei das erste Material (2) ferner mindestens ein Laschenelement (10) und mindestens eine Öffnung (11) aufweist, durch die das jeweilige Laschenelement (10) hindurchtreten kann.

5. Verfahren nach Anspruch 4, wobei die mindestens eine Öffnung (11) ein Schlitz ist.

6. Verfahren nach Anspruch 4 oder 5, wobei die zylindrische Konfiguration durch Biegen des ersten Materials (2) in die zylindrische Konfiguration und Fixieren der zylindrischen Konfiguration durch Hindurchführen des mindestens einen Laschenelements (10) durch die jeweilige mindestens eine Öffnung (11) zur Bildung einer Laschen-SchlitzVerbindung erhalten wird.

7. Verfahren nach einem der Ansprüche 4 bis 6, wobei das mindestens eine Laschenelement (10) einen mittleren Abschnitt aufweist, der mit einem Kopfabschnitt verbunden ist, und wobei der mittlere Abschnitt eine kleinere Größe als der Kopfabschnitt hat und die Größe des mittleren Abschnitts kleiner oder gleich der Größe der jeweiligen mindestens einen Öffnung (11) ist.

8. Verfahren nach einem der vorhergehenden Ansprüche, wobei das erste Material (2) außerdem mindestens einen Befestigungsabschnitt (15) aufweist, der aus dem Hauptkörper (14) herausragt.

9. Verfahren nach Anspruch 8, wobei der mindestens eine Befestigungsabschnitt (15) an einem Stent (1), vorzugsweise an mindestens einem mechanischen Befestigungselement (17, 18, 19) des Stents, angebracht wird.

10. Biologische Ummantelung (3) für einen Stent aus einem biologischen Material (23), vorzugsweise erhalten durch ein Verfahren nach einem der vorhergehenden Ansprüche, umfassend einen Hauptkörper (14) aus dem biologischen Material in einer zylindrischen Konfiguration mit einem ersten Zylinderdurchmesser; und mindestens eine Falte in dem Hauptkörper, wobei die mindestens eine Falte so konfiguriert ist, dass sie sich bei radialer Dilatation des biologischen Materials zumindest teilweise entfaltet, um eine Vergrößerung der zylindrischen Konfiguration auf einen zweiten Zylinderdurchmesser zu ermöglichen, **dadurch gekennzeichnet, dass** das biologische Material vernetzt ist.

11. Biologische Ummantelung (3) nach Anspruch 10, die ferner mindestens ein Laschenelement (10) und mindestens eine Öffnung (11) umfasst, durch die das jeweilige Laschenelement (10) hindurchgeführt werden kann, und wobei das mindestens eine Laschenelement (10) durch die jeweilige mindestens eine Öffnung (11) hindurchgeführt wird, um eine Laschen- und Schlitzverbindung zu bilden.

12. Biologische Ummantelung (3) nach Anspruch 11, wobei das Laschenelement (10) relativ zur Öffnung entlang einer Durchgangsrichtung beweglich ist.

13. Die biologische Ummantelung (3) nach einem der Ansprüche 10 bis 12, wobei das biologische Material (23) außerdem mindestens einen Befestigungsabschnitt (15) umfasst, der aus dem Hauptkörper (14) herausragt und an einem Stent befestigt werden kann.

14. Die biologische Ummantelung (3) nach einem der Ansprüche 10 bis 13, wobei das biologische Material (23) ein kollagenhaltiges Gewebe, vorzugsweise Perikardgewebe, ist.

15. Vorrichtung (20) zur Herstellung einer biologischen Abdeckung (3) gemäß dem Verfahren nach einem der Ansprüche 1 bis 9, umfassend ein Gegenelement (21) aus einem Polymerschaum, ein Druckerzeugungsmittel (22), das in ein inneres Lumen (Lu) des zylindrischen biologischen Materials (23) eingeführt werden kann, um das biologische Material (23) auf das Gegenelement (21) zu pressen, wobei das Druckerzeugungsmittel (24) einen anisotropen Druck auf das zylindrische biologische Material (23) ausüben kann.

## Revendications

1. Procédé de production d'un revêtement biologique pour un stent (3) comprenant les étapes consistant à :
fournir un premier matériau (2) ayant un corps principal (14) dans une configuration cylindrique et ayant au moins un pli dans le premier matériau et ayant un second matériau (5) agencé à l'intérieur de l'au moins un pli, dans lequel le premier matériau (2) est un matériau biologique (23) ;
presser le premier matériau (2) et le second matériau (5) sur un contre-élément (21) agencé à l'extérieur de la configuration cylindrique ; et
appliquer un agent de réticulation au premier matériau (2), **caractérisé en ce que** le premier matériau (2) comprend des groupements réticulables qui peuvent être réticulés par utilisation de l'agent de réticulation et dans lequel le second matériau (2) est un matériau qui ne peut pas être réticulé au premier matériau (2) par utilisation de l'agent de réticulation.

2. Procédé selon la revendication 1, dans lequel le premier matériau (2) comprend plusieurs plis.

3. Procédé selon la revendication 1 ou 2, dans lequel le second matériau (2) est fabriqué en un polymère organique.

4. Procédé selon l'une quelconque des revendications précédentes, dans lequel le premier matériau (2) comprend en outre au moins un élément de languette (10) et au moins une ouverture (11) pour que l'élément de languette (10) respectif passe à travers.

5. Procédé selon la revendication 4, dans lequel l'au moins une ouverture (11) est une fente.

6. Procédé selon la revendication 4 ou 5, dans lequel la configuration cylindrique est obtenue en pliant le premier matériau (2) dans la configuration cylindrique et en fixant la configuration cylindrique en passant l'au moins un élément de languette (10) à travers l'au moins une ouverture (11) respective pour former au moins une liaison de type languette-fente.

7. Procédé selon l'une quelconque des revendications 4 à 6, dans lequel l'au moins un élément de languette (10) comprend une partie médiane reliée à une partie de tête, et dans lequel la partie médiane a une taille inférieure à celle de la partie de tête et la taille de la partie médiane est inférieure ou égale à la taille de l'au moins une ouverture (11) respective.

8. Procédé selon l'une quelconque des revendications précédentes, dans lequel le premier matériau (2) comprend en outre au moins une partie d'attachement (15) faisant saillie depuis le corps principal (14).

9. Procédé selon la revendication 8, dans lequel l'au moins une partie d'attachement (15) est attachée à un stent (1), préférentiellement à l'au moins un élément de fixation mécanique (17, 18, 19) du stent.

10. Revêtement biologique (3) destiné à un stent fabriqué en un matériau biologique (23), préférentiellement obtenu par un procédé selon l'une quelconque des revendications précédentes, comprenant un corps principal (14) du matériau biologique dans une configuration cylindrique ayant un premier diamètre de cylindre ; et au moins un pli dans le corps principal, dans lequel l'au moins un pli est conçu pour se déplier au moins partiellement lors d'une dilatation radiale du matériau biologique afin de permettre l'agrandissement de la configuration cylindrique jusqu'à un second diamètre de cylindre, **caractérisé en ce que** le matériau biologique est réticulé.

11. Revêtement biologique (3) selon la revendication 10, comprenant en outre au moins un élément de languette (10) et au moins une ouverture (11) pour que l'élément de languette (10) respectif passe à travers, et dans lequel l'au moins un élément de languette (10) passe à travers l'au moins une ouverture (11) respective pour former une liaison de type languette-fente.

12. Revêtement biologique (3) selon la revendication 11, dans lequel l'élément de languette (10) peut être déplacé par rapport à l'ouverture le long d'une direction de passage à travers.

13. Revêtement biologique (3) selon l'une quelconque des revendications 10 à 12, dans lequel le premier matériau (23) comprend en outre au moins une partie d'attachement (15) faisant saillie depuis le corps principal (14) et qui peut être attachée à un stent.

14. Revêtement biologique (3) selon l'une quelconque des revendications 10 à 13, dans lequel le matériau biologique (23) est un tissu contenant du collagène, préférentiellement un tissu péricardique.

15. Dispositif (20) de production d'un revêtement biologique (3) selon le procédé de l'une quelconque des revendications 1 à 9, comprenant un contre-élément (21) fabriqué en une mousse polymère, un moyen de génération de pression (22) adapté pour être introduit dans une lumière intérieure (Lu) du matériau biologique cylindrique (23) afin de presser le matériau biologique (23) sur le contre-élément (21), dans lequel le moyen de génération de pression (24) est adapté pour appliquer une pression anisotrope au matériau biologique cylindrique (23).
